# EUROPÄISCHE PATENTSCHRIFT

(11) **EP 3 017 795 B1**
(45) Veröffentlichungstag und Bekanntmachung des Hinweises auf die Patenterteilung: **05.07.2017**
(21) Anmeldenummer: 15190732.6
(22) Anmeldetag: 21.10.2015
(51) Int. Cl.: A61F 9/02, A61F 9/04

(54) **AUGENSCHUTZ-BRILLE**
EYE PROTECTION GOOGLES
LUNETTES DE PROTECTION

(30) Priorität: 31.10.2014 DE 102014222329
(43) Veröffentlichungstag der Anmeldung: 11.05.2016
(73) Patentinhaber: Laservision GmbH & Co. KG, 90766 Fürth (DE)
(72) Erfinder: Penninger, Christian, 90489 Nürnberg (DE); Horn, Lars, 22763 Hamburg (DE); Fröhlich, Thomas, 91207 Lauf an der Pegnitz (DE)
(74) Vertreter: Rau, Schneck & Hübner Patentanwälte Rechtsanwälte PartGmbB

(56) Entgegenhaltungen:
- DE-B3-102005 058 888
- FR-A- 1 393 280
- US-A- 5 927 281

## Beschreibung

Die vorliegende Patentanmeldung nimmt die Priorität der deutschen Patentanmeldung DE 10 2014 222 329.6 in Anspruch, deren Inhalt durch Bezugnahme hierin aufgenommen wird.

Die Erfindung betrifft eine Augenschutz-Brille zum Schutz einer zu behandelnden Person vor elektromagnetischer Strahlung, insbesondere vor Laserstrahlung, gemäß dem Oberbegriff des Anspruchs 1. Ferner richtet sich die Erfindung auf eine Anordnung als Bestandteil einer derartigen Augenschutz-Brille.

Durch medizinische Laserbehandlungen können bei Personen Falten oder Hautanomalien, wie Altersflecken und Warzen, behandelt werden. Dabei müssen die Augen der zu behandelnden Person und die Augen der behandelnden Person vor der auftretenden Laserstrahlung geschützt werden.

Zum Schutz der Augen der zu behandelnden Person kann bekanntlich beispielsweise eine Augapfel-Brille herangezogen werden, die zwei Halbschalen zum Bedecken der Augen umfasst. Die Halbschalen sind über einen Verbindungssteg miteinander verbunden und sind über ein Kopfband an dem Kopf der zu behandelnden Person haltbar. Eine gattungsgemäße Augapfel-Brille ist beispielsweise aus der DE 10 2005 058 888 B3 bekannt. Nachteilig bei derartigen Augapfel-Brillen ist, dass deren Schutzwirkung oftmals nicht ausreichend ist. Ferner werden diese häufig von der diese tragenden Person als unangenehm empfunden.

Aus der US 6,081,934 ist eine Augenschutz-Brille zum Schutz einer zu behandelnden Person bei einer Laserbehandlung im Gesicht bekannt. Die Augenschutz-Brille umfasst zwei Augenschutz-Kappen. Jede Augenschutz-Kappe trägt einen umlaufenden Befestigungsflansch, der ausgebildet ist, um ein umlaufendes Haut-Dichtelement zu tragen. Das Haut-Dichtelement besteht aus einem weichen, flexiblen Material, wie Gummi. An jeder Augenschutz-Kappe ist ferner ein Nasenbrücken-Anschlussteil angeordnet. Ein verstellbares Nasenbrücken-Band ist an den Nasenbrücken-Anschlussteilen befestigt und verbindet die Augenschutz-Kappen. Diese Augenschutz-Brille ist aufwendig in ihrer Fertigung. Ferner stört das Nasenbrücken-Band im Allgemeinen bei einer Laserbehandlung im Gesicht.

Die US 5,918,600 offenbart eine Augenschutz-Vorrichtung mit zwei separaten Augenschirmen, die Laserstrahlung blockieren. Jeder Augenschirm trägt einen Haken, an dem ein Ende einer Nasenbrücken-Feder befestigbar ist.

Die US 5,927,281 offenbart eine Brille zum Verhindern von Keratitis. Die Brille umfasst Augenschutzteile und ein verstellbares Nasenband, das die Augenschutzteile miteinander verbindet. Ferner sind Kissenelemente vorgesehen, die an den Augenschutzteilen zur Anlage an dem Gesicht eines Trägers angeordnet sind.

Aus der FR 1 393 280 A ist eine Augenschutzvorrichtung mit zwei Augenschalen bekannt, die über einen Nasensteg miteinander verbunden sind. Der Nasensteg greift in Öffnungen an Ansätzen ein, die fest mit den Augenschalen verbunden sind.

Der Erfindung liegt die Aufgabe zugrunde, eine Augenschutz-Brille bereitzustellen, die einerseits einen besonders hohen Schutz vor elektromagnetischer Strahlung bietet und andererseits äußerst bequem zu tragen ist. Eine entsprechende Anordnung als Bestandteil einer Augenschutz-Brille soll ebenfalls geschaffen werden.

Diese Aufgabe wird erfindungsgemäß durch die in den unabhängigen Ansprüchen 1 und 13 angegebenen Merkmale gelöst. Der Kern der Erfindung liegt darin, dass die Augenschutz-Kappen zum Bedecken der Augen der zu behandelnden Person jeweils einen Grund-Schutzkörper und einen Decken-Schutzkörper umfassen, die in zusammengesetztem Zustand der jeweiligen Augenschutz-Kappe in lösbarer Verbindung miteinander stehen. Die Augenschutz-Kappen sind also jeweils mehrteilig, insbesondere zweiteilig, ausgeführt. Eine besonders gründliche Reinigung der Augenschutz-Kappen ist demnach möglich. Ferner sind die Schutzkörper so gezielt an die jeweiligen Erfordernisse, insbesondere in Sachen Schutzwirkung und/oder Tragekomfort, anpassbar.

Die unlösbare, einstückige Verbindung zwischen dem Decken-Schutzkörper und dem Verbindungssteg führt zu einer verliersicheren Verbindung zwischen diesen. Die einstückige Verbindung zwischen den Decken-Schutzkörpern und dem Verbindungssteg erlaubt eine besonders einfache und kostengünstige Herstellung derselben. Es ist von Vorteil, wenn die Decken-Schutzkörper und der Verbindungssteg aus einem identischen Material gebildet sind.

Bei der erfindungsgemäßen Augenschutz-Brille ist der Verbindungssteg besonders einfach bzw. gezielt verlagerbar, um insbesondere einen störungsfreien Zugang zu dem zu behandelnden Bereich der zu behandelnden Person zu erhalten.

Vorzugsweise ist jeder Grund-Schutzkörper schalen- bzw. kappenartig. Günstigerweise ist also jeder Grund-Schutzkörper als hohler Formkörper ausgeführt. Die Grund-Schutzkörper sind vorzugsweise metallfrei bzw. aus einem desinfizierbaren, insbesondere autoklavierbaren, Material gebildet.

Es ist von Vorteil, wenn jeder Grund-Schutzkörper eine Seitenwand hat, die vorteilhafterweise in einer Umfangsrichtung geschlossen ist. Die Seitenwand bietet insbesondere Schutz vor seitlicher elektromagnetischer Strahlung.

Es ist zweckmäßig, wenn jeder Grund-Schutzkörper eine Decke hat. Die Decke schließt sich günstigerweise in einem Endbereich an die jeweilige Seitenwand an und verschließt einseitig den Grund-Schutzkörper. Sie ist demnach günstigerweise im Wesentlichen gegenüberliegend zu dem jeweiligen freien Rand an der jeweiligen Seitenwand angeordnet. Die Decke bietet insbesondere Schutz vor frontaler elektromagnetischer Strahlung. Die Seitenwand und die Decke von jeder Augenschutz-Kappe sind günstigerweise einteilig bzw. einstückig miteinander verbunden. Es ist zweckmäßig, wenn diese aus einem identischen Material gebildet sind.

Günstigerweise ist durch jeden Grund-Schutzkörper ein Innenraum nach seitlich außen räumlich begrenzt. Es ist zweckmäßig, wenn sich der Innenraum in einer Blickrichtung der Augenschutz-Brille zumindest bereichsweise verjüngt. Die Innenräume sind vorzugsweise über Gesichts-Öffnungen offen bzw. zugänglich.

Es ist zweckmäßig, wenn sich jeder Grund-Schutzkörper in einer Breitenrichtung der Augenschutz-Brille, also in Richtung eines Augenabstands der zu behandelnden Person, längs erstreckt.

Es ist von Vorteil, wenn jeder Decken-Schutzkörper plattenartig bzw. plättchenartig ausgebildet ist. Die Decken-Schutzkörper sind vorzugsweise metallfrei. Günstigerweise ist jeder Decken-Schutzkörper in zusammengesetztem Zustand der Augenschutz-Brille außenseitig benachbart zu der jeweiligen Decke angeordnet. Durch jeden Decken-Schutzkörper ist der Schutz vor elektromagnetischer Strahlung weiter verbesserbar bzw. steigerbar. Die Decken-Schutzkörper bieten insbesondere zusätzlichen Schutz vor frontaler elektromagnetischer Strahlung. Sie bilden günstigerweise Zusatz-Schutzkörper.

Die Schutzwirkung der Augenschutz-Brille bzw. der Augenschutz-Kappen ist vergleichbar mit Metall-basierten Augenschutz-Brillen und demnach wesentlich höher als die von planaren Klebepads.

Die Augenschutz-Kappen sind vorzugsweise als hohle Formkörper ausgebildet. Sie sind günstigerweise undurchsichtig. Die Augenschutz-Kappen sind vorzugsweise metallfrei bzw. aus einem desinfizierbaren, insbesondere autoklavierbaren, Material gebildet.

Der Verbindungssteg hält die Augenschutz-Kappen zusammen. Er ist günstigerweise als Brücke ausgeführt.

Es ist zweckmäßig, wenn die Augenschutz-Kappen über eine Kopf-Halteeinrichtung an dem Kopf der zu behandelnden Person örtlich festlegbar sind. Die Kopf-Halteeinrichtung kann direkt oder indirekt an den Augenschutz-Kappen angeordnet sein. Sie ist beispielsweise als Kopfband, Bügel-Anordnung oder dergleichen ausgeführt. Sie kann einteilig oder mehrteilig sein.

Es ist von Vorteil, wenn die Anordnung gemäß Anspruch 13 einteilig bzw. einstückig ausgeführt ist. Sie ist vorzugsweise aus einem einheitlichen Material gebildet.

Weitere vorteilhafte Ausgestaltungen der Erfindung sind in den Unteransprüchen angegeben.

Die Augenschutz-Brille gemäß Unteranspruch 2 erfüllt in einfacher Weise die Anforderung, die an moderne Augenschutz-Brillen gestellt werden. Sie ist besonders gut beispielsweise in Sachen Härte und/oder (Laser-)Schutzwirkung einstellbar.

Die Grund-Schutzkörper gemäß Unteranspruch 3 sind imstande, sich besonders gut an das Gesicht bzw. die Augenpartien der zu behandelnden Person anzupassen. Es ist so im Wesentlichen besonders sicher verhinderbar, dass Spalte zwischen dem jeweiligen Grund-Schutzkörper und dem Gesicht der zu behandelnden Person auftreten, wenn die Augenschutz-Brille bestimmungsgemäß getragen wird. Solche Spalte sind gefährlich, da durch diese elektromagnetische Strahlung ungehindert zu den Augen der zu behandelnden Person gelangen kann. Die nachgiebige Ausgestaltung der Grundschutz-Körper, die im Einsatz an dem Gesicht der zu behandelnden Person anliegen, führt zu einem äußerst hohen Tragekomfort.

Gemäß Unteranspruch 4 sind die Decken-Schutzkörper und/oder der Verbindungssteg im Wesentlichen formstabil bzw. starr. In einem gewissen Maße ist insbesondere der Verbindungssteg manuell verformbar, sodass beispielsweise der Abstand der Augenschutz-Kappen zueinander veränderbar ist. Separate Gelenke, Scharnier oder dergleichen sind dafür nicht erforderlich. Die Augenschutz-Kappen sind so einfach an die Augen der zu behandelnden Person bzw. an deren Abstand zueinander anpassbar.

Durch die Ausgestaltung gemäß Unteranspruch 5 ist eine besonders sichere und spaltlose Verbindung zwischen dem Decken-Schutzkörper und dem Grund-Schutzkörper erzielbar. Eine derartige Verbindung ist einfach herstellbar und auch wieder auflösbar.

Es ist von Vorteil, wenn das mindestens eine Formschlussmittel gemäß dem Unteranspruch 6 vollständig umlaufend ausgeführt ist bzw. in einer Umfangsrichtung geschlossen ist. Die Nut ist günstigerweise im Querschnitt rechteckig. Vorzugsweise ist sie nach seitlich außen offen. Das mindestens eine Gegen-Formschlussmittel ist günstigerweise vollständig umlaufend bzw. in einer Umfangsrichtung geschlossen. Es ist vorzugsweise im Querschnitt rechteckig. Günstigerweise greift das mindestens eine Gegen-Formschlussmittel von seitlich außen in das mindestens eine Formschlussmittel formschlüssig ein. Eine andere bzw. umgekehrte Anordnung ist alternativ möglich.

Die Ausgestaltung gemäß Unteranspruch 7 erlaubt ein im Wesentlichen störungsfreies Behandeln der zu behandelnden Person durch die behandelnde Person. Es ist so möglich, den Verbindungssteg so zu verlagern, dass er einen freien Zugang zu einem zu behandelnden Bereich der zu behandelnden Person erlaubt. Wenn beispielsweise ein Stirnbereich der zu behandelnden Person zu behandeln ist, wird der Verbindungssteg aus dem Stirnbereich, beispielsweise in einen Nasenbereich der zu behandelnden Person, verlagert bzw. verschwenkt. Wenn beispielsweise ein Nasenbereich der zu behandelnden Person zu behandeln ist, wird der Verbindungssteg aus dem Nasenbereich, beispielsweise in einen Stirnbereich der zu behandelnden Person, verlagert bzw. verschwenkt. Es ist von Vorteil, wenn der Verbindungssteg in mindestens zwei stabile Positionen verlagerbar bzw. verschwenkbar ist. Alternativ ist der Verbindungssteg stufenlos verlagerbar. Gemäß einer bevorzugten Ausführungsform ist der Verbindungssteg im Wesentlichen in seiner Gesamtheit verlagerbar. Alternativ ist er nur bereichsweise, günstigerweise mit einem Mittenbereich, verlagerbar.

Die Ausgestaltung gemäß Unteranspruch 8 führt zu einer Augenschutz-Brille, die besonders gut an die zu behandelnde Person bzw. an deren Augen(abstand) anpassbar ist. Diese Augenschutz-Brille bietet einen besonders hohen Tragekomfort und ist äußerst sicher.

Die Augenschutz-Brille gemäß Unteranspruch 9 erlaubt wieder eine gute Zugänglichkeit für die behandelnde Person zu den zu behandelnden Bereichen der zu behandelnden Person. In Abhängigkeit der Tragestellung der Augenschutz-Brille erstreckt sich der Verbindungssteg günstigerweise entweder in einer ersten Höhenrichtung der Augenschutz-Brille oder in einer zu der ersten Höhenrichtung gegensinnigen zweiten Höhenrichtung. Vorzugsweise ist bei dieser Ausführungsform der Verbindungssteg gegenüber den Augenschutz-Kappen unbeweglich.

Die Ausgestaltung gemäß dem Unteranspruch 10 ergibt eine Augenschutz-Brille, die besonders gut an der zu behandelnden Person örtlich festlegbar ist. Es ist von Vorteil, wenn jeder Kopplungskörper bügelartig ist bzw. gekrümmt verläuft. Günstigerweise ist in jeden Kopplungskörper ein Kopfband zum Umlaufen des Kopfs der zu behandelnden Person einhängbar.

Die Ausgestaltung gemäß Unteranspruch 11 führt zu einer verliersicheren Verbindung zwischen dem Decken-Schutzkörper und dem zugeordneten Kopplungskörper. Es ist von Vorteil, wenn der Decken-Schutzkörper und der zugehörige Kopplungskörper aus einem identischen Material gebildet sind.

Die Ausgestaltung gemäß Unteranspruch 12 erlaubt eine besonders einfache und gezielte Verstellung der Kopplungskörper in einer Höhenrichtung. Die Augenschutz-Brille ist so äußerst gut an die zu behandelnde Person anpassbar.

Die in den Unteransprüchen 2 bis 12 angegebenen bevorzugten Ausgestaltungen können auch Gegenstand des Anspruchs 13 sein.

Nachfolgend wird unter Bezugnahme auf die beigefügte Zeichnung eine bevorzugte Ausführungsform der Erfindung beispielhaft beschrieben. Dabei zeigen:
- Fig. 1: eine Explosionsansicht einer erfindungsgemäßen Augenschutz-Brille,
- Fig. 2: die in Fig. 1 dargestellte Augenschutz-Brille in einem zusammengesetzten Zustand,
- Fig. 3: eine Ansicht von vorne, die die Augenschutz-Kappen und einen Verbindungssteg sowie die Kopplungskörper der in den Fig. 1 und 2 veranschaulichten Augenschutz-Brille zeigt,
- Fig. 4: einen Längsschnitt durch eine Augenschutz-Kappe gemäß der in Fig. 3 dargestellten Schnittlinie IV-IV,
- Fig. 5: eine Explosionsansicht der in Fig. 4 veranschaulichten Augenschutz-Kappe, und
- Fig. 6: die in Fig. 2 dargestellte, zusammengesetzte Augenschutz-Brille in einem getragenen Zustand, wobei sich der Verbindungssteg in einer im Vergleich mit Fig. 3 anderen, unteren Position befindet.

Eine Augenschutz-Brille bzw. Augapfel-Brille umfasst zwei Augenschutz-Kappen 1, einen die Augenschutz-Kappen 1 miteinander verbindenden

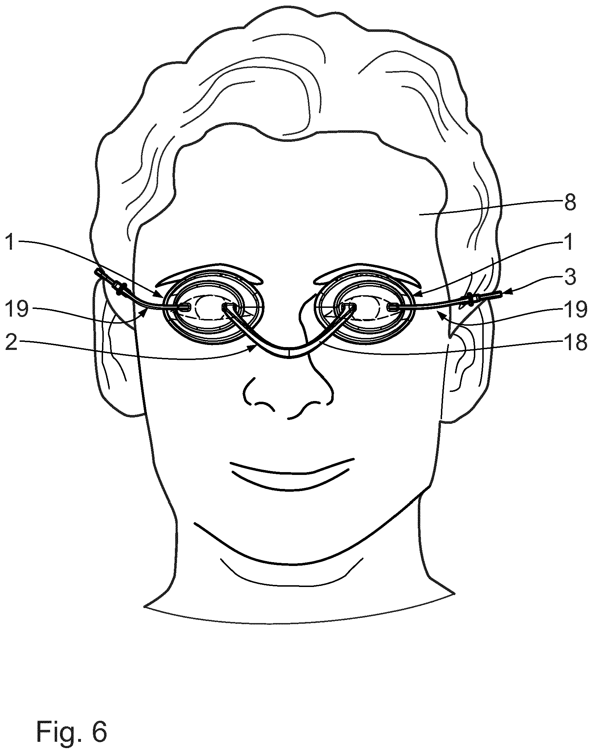

Verbindungssteg 2 und ein mit den Augenschutz-Kappen 1 in Verbindung stehendes Kopfband 3. Die Augenschutz-Brille ist bezüglich einer Symmetrieebene E symmetrisch ausgeführt, die sich mittig zwischen den Augenschutz-Kappen 1 erstreckt. Wenn die Augenschutz-Brille bestimmungsgemäß entsprechend Fig. 6 getragen wird, bedecken die Augenschutz-Kappen 1 vollständig die zwei Augen der zu behandelnden Person 8. Für jedes Auge ist eine eigene Augenschutz-Kappe 1 vorgesehen.

Nachdem die Augenschutz-Kappen 1 identisch ausgeführt sind, wird nachfolgend nur auf eine Augenschutz-Kappe 1 eingegangen.

Jede Augenschutz-Kappe 1 umfasst einen Grund-Schutzkörper 4 und einen mit diesem Grund-Schutzkörper 4 lösbar verbindbaren Decken-Schutzkörper 5. Die Grund-Schutzkörper 4 sind im Wesentlichen flexibel bzw. nachgiebig, während die Decken-Schutzkörper 5 im Wesentlichen formstabil ausgeführt sind. In zusammengesetztem Zustand der Augenschutz-Kappen 1 ist jeder Decken-Schutzkörper 5 mit dem zugeordneten Grund-Schutzkörper 4 lösbar, formschlüssig verbunden.

Jeder Grund-Schutzkörper 4 hat eine umfangsseitig geschlossene Seitenwand 6 mit einem fußseitigen, freien Rand 7 zur Anlage an dem Gesicht der zu behandelnden Person 8. Beabstandet zu dem jeweiligen freien Rand 7 hat jeder Grund-Schutzkörper 4 eine Decke 9, die den Grund-Schutzkörper 4 in einer Blickrichtung 10 der Augenschutz-Brille verschließt. Die Decken 9 sind jeweils plattenartig ausgeführt und erstrecken sich im Wesentlichen senkrecht zu der Blickrichtung 10. Gegenüberliegend zu den freien Rändern 7 ist an jeder Seitenwand 6 ein Formschluss-Steg 11 angeordnet, der umfangsseitig geschlossen ist und von der jeweiligen Seitenwand 6 nach innen vorspringt.

Der Formschluss-Steg 11, die Seitenwand 6 und die Decke 9 sind bei der jeweiligen Augenschutz-Kappe 1 jeweils einstückig miteinander verbunden. Sie sind aus einem identischen Material, vorzugsweise Silikon, gebildet. Durch die Seitenwand 6 und die Decke 9 ist bei jeder Augenschutz-Kappe 1 jeweils ein Innenraum 12 räumlich begrenzt, der vorzugsweise im Querschnitt kreisförmig, elliptisch oder oval ist.

Jeder Decken-Schutzkörper 5 weist eine umfangsseitig geschlossene Formschluss-Nut 13 auf, die nach seitlich außen offen und komplementär zu dem Formschluss-Steg 11 ausgebildet ist. Jeder Decken-Schutzkörper 5 hat benachbart zu der jeweiligen Formschluss-Nut 13 einen äußeren, nach seitlich außen vorspringenden Überstand 14, der umfangsseitig geschlossen ausgeführt ist und die jeweilige Formschluss-Nut 13 in der Blickrichtung 10 begrenzt.

Der Verbindungssteg 2 ist einstückig mit den Decken-Schutzkörpern 5 verbunden. Er ist bogenförmig gekrümmt und schließt sich jeweils an eine Außenfläche 15 des jeweiligen Decken-Schutzkörpers 5 an. Der Verbindungssteg 2 schließt sich insbesondere in einem seitlich inneren Bereich des jeweiligen Decken-Schutzkörpers 5 an diesen an. Er hat benachbart zu dem jeweiligen Decken-Schutzkörper 5 einen in einer Breitenrichtung 16 der Augenschutz-Brille, also einen in Richtung eines Augenabstands der zu behandelnden Person, längs erstreckenden, rechteckigen Querschnitt. Der Verbindungssteg 2 hat dort in der Breitenrichtung 16 jeweils eine Breite B und senkrecht dazu in einer Höhenrichtung 17 der Augenschutz-Brille eine Höhe H. Es gilt: 1,2 ≤ B/H ≤ 5, bevorzugter 1,8 ≤ B/H ≤ 4.

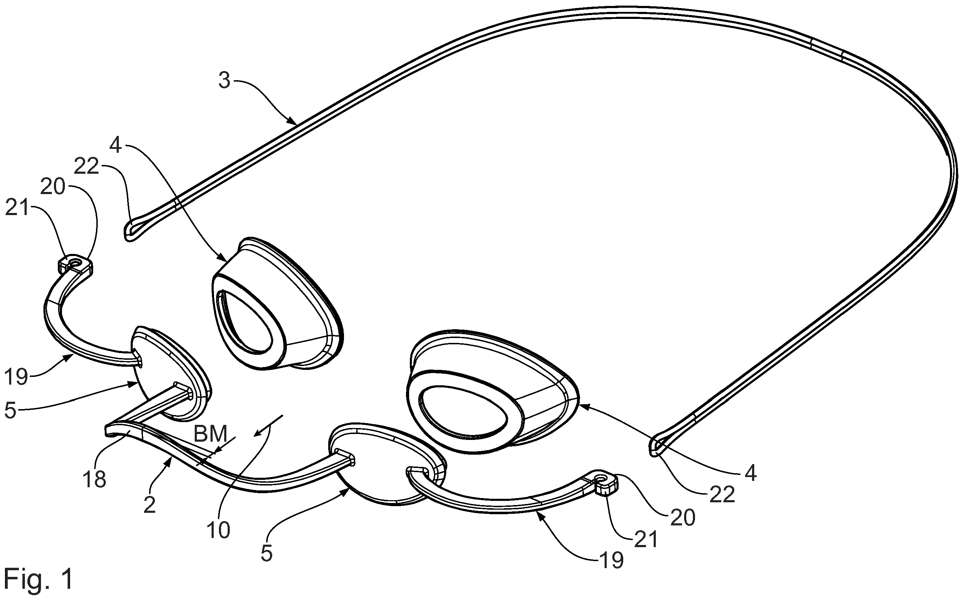

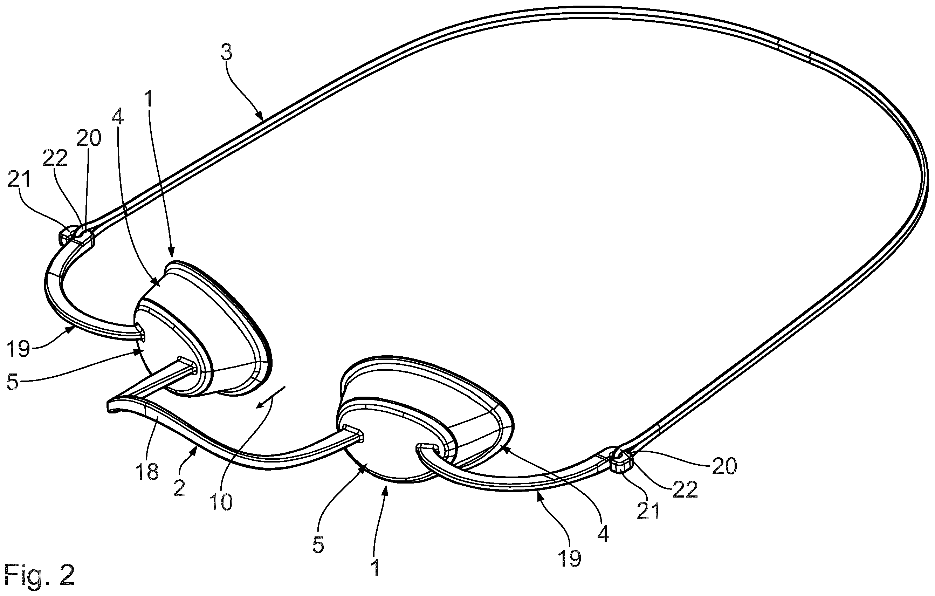

Der Verbindungssteg 2 hat grundsätzlich eine gleichbleibende Querschnittsform. Von den Decken-Schutzkörpern 5 weglaufend ändert sich jedoch die Orientierung des Querschnitts des Verbindungsstegs 2. Mittig zwischen den Decken-Schutzkörpern 5 ist der Querschnitt des Verbindungsstegs 2 um etwa 90° gegenüber dem Querschnitt des Verbindungsstegs 2 bei den Decken-Schutzkörpern 5 gedreht. Der Verbindungssteg 2 hat somit in einem Mittenbereich 18 zwischen den Decken-Schutzkörpern 5 eine Höhe HM in der Höhenrichtung 17 und eine Breite BM in der Breitenrichtung 16. Es gilt: 1,2 ≤ HM/BM ≤ 5, insbesondere 1,8 ≤ HM/BM ≤ 4. Von den Decken-Schutzkörpern 5 verläuft der Verbindungssteg 2 bei seiner Stellung gemäß Fig. 1 bis 3 in der Höhenrichtung 17 nach oben.

An jeden Decken-Schutzkörper 5 schließt sich außerdem ein armartiger Kopplungskörper 19 an, der jeweils bogenförmig bzw. gekrümmt ist. Jeder Kopplungskörper 19 schließt sich an die Außenfläche 15 in einem seitlich äußeren Bereich an. Von dem jeweiligen Decken-Schutzkörper 5 springt jeder Kopplungskörper 19 zunächst nach seitlich außen vorne vor und läuft dann bogenförmig nach hinten.

Benachbart zu den Decken-Schutzkörpern 5 hat jeder Kopplungskörper 19 eine sich längs der Breitenrichtung 16 erstreckende Breite BK und eine senkrecht zu dieser in der Höhenrichtung 17 erstreckende Höhe HK. Es gilt: 1,2 ≤ BK/HK ≤ 5, insbesondere 1,8 ≤ BK/HK ≤ 4.

Jeder Kopplungskörper 19 hat an seinem freien Ende 20 einen Haltehaken 21. Das Kopfband 3 ist in zusammengesetztem Zustand der Augenschutz-Brille mit endseitig angeordneten Schlaufen 22 in die Haltehaken 21 lösbar eingehängt.

Nachfolgend wird die Augenschutz-Brille in zusammengesetztem Zustand detaillierter beschrieben.

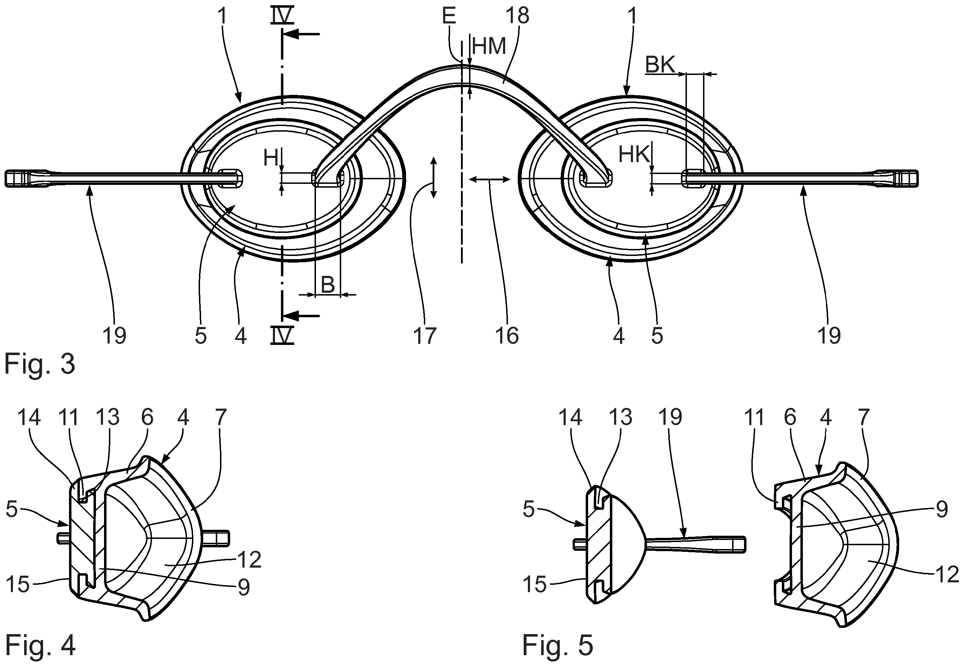

Wie insbesondere aus Fig. 4 hervorgeht, greifen in zusammengesetztem Zustand der Augenschutz-Brille bzw. der Augenschutz-Kappen 1 die Formschluss-Stege 11 von seitlich außen in die dazu komplementären Formschluss-Nuten 13 ein, wodurch die Decken-Schutzkörper 5 formschlüssig lösbar an den Grund-Schutzkörpern 4 örtlich festgelegt werden. Beim Einfügen bzw. Einsetzen der Decken-Schutzkörper 5 in die Grund-Schutzkörper 4 schnappen die Formschluss-Stege 11 in die Formschluss-Nuten 13 ein. Jeder Decken-Schutzkörper 5 erstreckt sich dann parallel und benachbart zu der zugehörigen Decke 9 und liegt günstigerweise auch zumindest bereichsweise, bevorzugter im Wesentlichen vollständig, an dieser außenseitig an. Jeder Überstand 14 schließt im Wesentlichen außenseitig bündig mit der jeweiligen Seitenwand 6 ab.

Zum Lösen der Decken-Schutzkörper 5 von den Grund-Schutzkörpern 4 werden die Formschluss-Stege 11 manuell außer Eingriff von den Formschluss-Nuten 13 gebracht.

Wie bereits erwähnt, bedecken die Augenschutz-Kappen 1 die Augen der zu behandelnden Person 8 vollständig und die Innenräume 12 sind zu den Augen hin offen, wenn die Augenschutz-Brille entsprechend Fig. 6 bestimmungsgemäß getragen wird. Die freien Ränder 7 liegen dabei an dem Gesicht der zu behandelnden Person 8 dicht an.

Wenn der Verbindungssteg 2 in der Höhenrichtung 17 gemäß Fig. 6 nach unten geklappt bzw. verschwenkt ist, befindet er sich über bzw. an der Nase der zu behandelnden Person 8, sodass deren Stirnbereich ungestört behandelbar ist.

Wenn der Verbindungssteg 2 in der Höhenrichtung 17 gemäß Fig. 1 nach oben geklappt bzw. verschwenkt ist, befindet sich dieser bei dem Stirnbereich der zu behandelnden Person 8, sodass deren Nase ungestört behandelbar ist.

Das Kopfband 3 läuft um den Kopf der zu behandelnden Person 8, sodass die Augenschutz-Brille an dem Kopf der zu behandelnden Person 8 gut fixiert ist.

## Patentansprüche

1. Augenschutz-Brille zum Schutz einer zu behandelnden Person (8) vor elektromagnetischer Strahlung, insbesondere vor Laserstrahlung, mit
a) zwei Augenschutz-Kappen (1), wobei jede Augenschutz-Kappe (1)
i) einen einen freien Rand (7) aufweisenden Grund-Schutzkörper (4) zum Schutz vor elektromagnetischer Strahlung umfasst, wobei der freie Rand (7) zur Anlage an dem Gesicht der zu behandelnden Person (8) bestimmt ist,
ii) einen mit dem jeweiligen Grund-Schutzkörper (4) in lösbarer Verbindung stehenden Decken-Schutzkörper (5) zum Schutz vor frontaler elektromagnetischer Strahlung umfasst, und
iii) einen Innenraum (12) räumlich begrenzt, und
b) einen die Decken-Schutzkörper (5) miteinander verbindenden Verbindungssteg (2),
**dadurch gekennzeichnet, dass**
c) die Decken-Schutzkörper (5) und der Verbindungssteg (2) unlösbar, einstückig, miteinander verbunden sind, und
d) der Verbindungssteg (2) zumindest benachbart zu den Augenschutz-Kappen (1) jeweils einen sich in einer Breitenrichtung (16) der Augenschutz-Brille längs erstreckenden Querschnitt zur Verlagerung des Verbindungsstegs (2) gegenüber den Augenschutz-Kappen (1) im Wesentlichen senkrecht zu einer Blickrichtung (10) der Augenschutz-Brille in einer Höhenrichtung (17) derselben aufweist,
i) wobei der sich zumindest benachbart zu den Augenschutz-Kappen (1) jeweils in der Breitenrichtung (16) der Augenschutz-Brille längs erstreckende Querschnitt des Verbindungsstegs (2) rechteckig ist,
ii) wobei der Verbindungssteg (2) dort in der Breitenrichtung (16) jeweils eine Breite (B) und senkrecht dazu in der Höhenrichtung (17) der Augenschutz-Brille eine Höhe (H) aufweist, wobei gilt: 1,2 ≤ B/H ≤ 5.

2. Augenschutz-Brille nach Anspruch 1, **dadurch gekennzeichnet, dass** die Grund-Schutzkörper (4) aus einem ersten Material und die Decken-Schutzkörper (5) aus einem sich von dem ersten Material, insbesondere in Härte und/oder Schutzwirkung, unterscheidenden zweiten Material gebildet sind.

3. Augenschutz-Brille nach Anspruch 1 oder 2, **dadurch gekennzeichnet, dass** die Grund-Schutzkörper (4) nachgiebig sind und insbesondere aus Silikon gebildet sind.

4. Augenschutz-Brille nach einem der vorherigen Ansprüche, **dadurch gekennzeichnet, dass** die Decken-Schutzkörper (5) und/oder der Verbindungssteg (2) im Wesentlichen formstabil sind/ist und insbesondere aus thermoplastischem Kunststoff, insbesondere Polyoxymethylen, gebildet sind/ist.

5. Augenschutz-Brille nach einem der vorherigen Ansprüche, **dadurch gekennzeichnet, dass** jeder Decken-Schutzkörper (5) mit dem zugehörigen Grund-Schutzkörper (4) formschlüssig und/oder kraftschlüssig lösbar verbunden ist.

6. Augenschutz-Brille nach einem der vorherigen Ansprüche, **dadurch gekennzeichnet, dass** jeder Decken-Schutzkörper (5) mindestens ein Formschlussmittel (13) und jeder Grund-Schutzkörper (4) mindestens ein Gegen-Formschlussmittel (11) zur formschlüssigen lösbaren Verbindung mit dem jeweiligen mindestens einen Formschlussmittel (13) umfasst, wobei vorzugsweise das mindestens eine Formschlussmittel (13) als umfangsseitige, insbesondere umlaufende, Nut (13) und das mindestens eine Gegen-Formschlussmittel (11) als, insbesondere umlaufender, Steg ausgeführt ist.

7. Augenschutz-Brille nach einem der vorherigen Ansprüche, **dadurch gekennzeichnet, dass** der Verbindungssteg (2) gegenüber den Augenschutz-Kappen (1) im Wesentlichen senkrecht zu der Blickrichtung (10) der Augenschutz-Brille in der Höhenrichtung (17) derselben zumindest teilweise verlagerbar ist.

8. Augenschutz-Brille nach einem der vorherigen Ansprüche, **dadurch gekennzeichnet, dass** der Verbindungssteg (2) beabstandet zu den Augenschutz-Kappen (1) einen sich in der Höhenrichtung (17) der Augenschutz-Brille längs erstreckenden, insbesondere rechteckigen, Querschnitt zur Veränderung des Abstands der Augenschutz-Kappen (1) zueinander aufweist.

9. Augenschutz-Brille nach einem der vorherigen Ansprüche, **dadurch gekennzeichnet, dass** sich der Verbindungssteg (2) zumindest bereichsweise in der Höhenrichtung (17) der Augenschutz-Brille erstreckt und die Augenschutz-Brille in einer ersten Stellung und einer zu der ersten Stellung um 180° gedrehten zweiten Kopfstellung tragbar ist.

10. Augenschutz-Brille nach einem der vorherigen Ansprüche, **dadurch gekennzeichnet, dass** an jedem Decken-Schutzkörper (5) ein, insbesondere nach seitlich außen vorspringender, günstigerweise im Wesentlichen formstabiler, Kopplungskörper (19) zum, insbesondere lösbaren, Halten einer Kopf-Halteeinrichtung (3) angeordnet ist.

11. Augenschutz-Brille nach Anspruch 10, **dadurch gekennzeichnet, dass** die Decken-Schutzkörper (5) und die Kopplungskörper (19) unlösbar, vorzugsweise einstückig, miteinander verbunden sind.

12. Augenschutz-Brille nach Anspruch 10 oder 11, **dadurch gekennzeichnet, dass** jeder Kopplungskörper (19) zumindest benachbart zu den Augenschutz-Kappen (1) einen sich in der Breitenrichtung (16) der Augenschutz-Brille längs erstreckenden, insbesondere rechteckigen, Querschnitt zur Verlagerung der Kopplungskörper (19) gegenüber den Augenschutz-Kappen (1) im Wesentlichen senkrecht zu der Blickrichtung (10) der Augenschutz-Brille in der Höhenrichtung (17) derselben aufweist.

13. Anordnung als Bestandteil einer Augenschutz-Brille nach einem der vorherigen Ansprüche, umfassend
a) zwei Decken-Schutzkörper (5), die mit zwei Grund-Schutzkörpern (4) zur Bildung von zwei Augenschutz-Kappen (1) lösbar verbindbar sind, und
b) einen mit den Decken-Schutzkörpern (5) in Verbindung stehenden Verbindungssteg (2),
c) wobei die Decken-Schutzkörper (5) und der Verbindungssteg (2) unlösbar, einstückig, miteinander verbunden sind,
d) wobei der Verbindungssteg (2) zumindest benachbart zu den Decken-Schutzkörpern (5) jeweils einen sich in einer Breitenrichtung (16) der Anordnung längs erstreckenden Querschnitt zur Verlagerung des Verbindungsstegs (2) gegenüber den Decken-Schutzkörpern (5) im Wesentlichen senkrecht zu einer Blickrichtung (10) der Anordnung in einer Höhenrichtung (17) derselben aufweist,
i) wobei der sich zumindest benachbart zu den Decken-Schutzkörpern (5) jeweils in der Breitenrichtung (16) der Anordnung längs erstreckende Querschnitt des Verbindungsstegs (2) rechteckig ist,
ii) wobei der Verbindungssteg (2) dort in der Breitenrichtung (16) jeweils eine Breite (B) und senkrecht dazu in der Höhenrichtung (17) der Anordnung eine Höhe (H) aufweist, wobei gilt: 1,2 ≤ B/H ≤ 5.

## Claims

1. Eye protection goggles for protecting a person (8) undergoing treatment from electromagnetic radiation, in particular from laser radiation, comprising
a) two eye protection caps (1), wherein each eye protection cap (1)
i) comprises a basic protection body (4), having a free edge (7), for protecting from electromagnetic radiation, wherein the free edge (7) is designed for placing on the face of the person (8) undergoing treatment,
ii) comprises a cover protection body (5), detachably connected to the respective basic protection body (4), for protecting from frontal electromagnetic radiation, and
iii) spatially delimits an inner chamber (12), and
b) a connecting web (2) connecting the cover protection bodies (5) with each other,
**characterized in that**
c) the cover protection bodies (5) and the connecting web (2) are connected non-detachably in one piece with each other, and
d) the connecting web (2) comprises at least adjacent to the eye protection caps (1) a respective cross section, extending longitudinally in a width direction (16) of the eye protection goggles, for displacing the connecting web (2) relative to the eye protection caps (1) essentially perpendicular to a line of vision (10) of the eye protection goggles in a vertical direction (17) thereof,
i) wherein the cross section of the connecting web (2), extending in each case longitudinally in the width direction (16) of the eye protection goggles at least adjacent to the eye protection caps (1), is rectangular,
ii) wherein the connecting web (2) has there, in the width direction (16), a respective width (B) and perpendicular thereto in a vertical direction (17) of the eye protection goggles a height (H), with 1,2 ≤ B/H ≤ 5.

2. Eye protection goggles according to claim 1, **characterized in that** the basic protection bodies (4) are made from a first material and the cover protection bodies (5) are made from a second material different from the first material, in particular in terms of hardness and/or protection effect.

3. Eye protection goggles according to claim 1 or 2, **characterized in that** the basic protection bodies (4) are flexible and are made in particular from silicone.

4. Eye protection goggles according to any one of the preceding claims, **characterized in that** the cover protection bodies (5) and/or the connecting web (2) is/are essentially dimensionally stable and is/are made in particular from thermoplastic material, in particular polyoxymethylene.

5. Eye protection goggles according to any one of the preceding claims, **characterized in that** each cover protection body (5) is connected detachably to the associated basic protection body (4) in a positive and/or force locking manner.

6. Eye protection goggles according to any one of the preceding claims, **characterized in that** each cover protection body (5) comprises at least one positive locking means (13) and each basic protection body (4) comprises at least one counter positive locking means (11) for releasable positive locking with the respective at least one positive locking means (13), wherein preferably the at least one positive locking means (13) is designed as a circumferential, in particular peripheral, groove (13) and the at least one counter positive locking means (11) is designed as an, in particular peripheral, web.

7. Eye protection goggles according to any one of the preceding claims, **characterized in that** the connecting web (2) is displaceable at least partly relative to the eye protection caps (1) essentially perpendicular to the line of vision (10) of the eye protection goggles in the vertical direction (17) thereof.

8. Eye protection goggles according to any one of the preceding claims, **characterized in that** the connecting web (2) spaced apart from the eye protection caps (1) has an, in particular rectangular, cross section extending longitudinally in the vertical direction (17) of the eye protection goggles for changing the distance between the eye protection caps (1).

9. Eye protection goggles according to any one of the preceding claims, **characterized in that** the connecting web (2) extends at least partly in the vertical direction (17) of the eye protection goggles and the eye protection goggles are wearable in a first position and a second head position rotated by 180° relative to the first position.

10. Eye protection goggles according to any one of the preceding claims, **characterized in that** on each cover protection body (5) a coupling body (19) for, in particular detachably, holding a head holding device (3) is arranged, wherein the coupling body (19) is advantageously dimensionally stable and in particular projects outwards laterally.

11. Eye protection goggles according to claim 10, **characterized in that** the cover protection bodies (5) and the coupling bodies (19) are joined non-detachably, preferably in one piece.

12. Eye protection goggles according to claim 10 or 11, **characterized in that** each coupling body (19) at least adjacent to the eye protection caps (1) has an, in particular rectangular, cross section extending longitudinally in the width direction (16) of the eye protection goggles for displacing the coupling bodies (19) relative to the eye protection caps (1) essentially perpendicular to the line of vision (10) of the eye protection goggles in the vertical direction (17) thereof.

13. Arrangement as a component of eye protection goggles according to any one of the preceding claims, comprising
a) two cover protection bodies (5) which are connectable detachably to two basic protection bodies (4) to form two eye protection caps (1), and
b) a connecting web (2) in connection with the cover protection bodies (5),
c) wherein the cover protection bodies (5) and the connecting web (2) are joined non-detachably in one piece,
d) wherein the connecting web (2) at least adjacent to the cover protection bodies (5) has a respective cross-section, extending in each case longitudinally in a width direction (16) of the arrangement, for displacing the connecting web (2) relative to the cover protection bodies (5) essentially perpendicular to a line of vision (10) of the arrangement in a vertical direction (17) thereof,
i) wherein the cross-section of the connecting web (2) extending in each case in the width direction (16) of the arrangement at least adjacent to the cover protection bodies (5) is rectangular,
ii) wherein the connecting web (2) has there, in the width direction (16), a respective width (B) and perpendicular thereto in the vertical direction (17) of the arrangement a height (H), with 1,2≤B/H≤5.

## Revendications

1. Lunettes de protection permettant de protéger une personne (8) à traiter contre un rayonnement électromagnétique, en particulier contre un rayonnement laser, comprenant
a) deux capuchons de protection oculaire (1), dans lesquelles chaque capuchon de protection oculaire (1)
i) comprend un corps de protection inférieur (4) comportant un bord libre (7) permettant une protection contre un rayonnement électromagnétique, dans lequel le bord libre (7) est destiné à être en appui contre le visage de la personne (8) à traiter,
ii) comprend un corps de protection supérieur (5) en liaison détachable avec le corps de protection inférieur (4) respectif permettant une protection contre un rayonnement électromagnétique frontal, et
iii) délimite dans l'espace un espace intérieur (12), et
b) un élément de liaison (2) reliant les corps de protection supérieurs (5) les uns aux autres,
**caractérisé en ce que**
c) les corps de protection supérieurs (5) et l'élément de liaison (2) sont reliés les uns aux autres d'une seule pièce et de manière non détachable, et
d) l'élément de liaison (2) présente au moins au voisinage des capuchons de protection oculaire (1) respectivement une section transversale s'étendant longitudinalement dans un sens de la largeur (16) des lunettes de protection pour le déplacement de l'élément de liaison (2) par rapport aux capuchons de protection oculaire (1) sensiblement perpendiculairement à une direction du regard (10) des lunettes de protection dans un sens de la hauteur (17) de celles-ci,
i) dans lesquelles la section transversale de l'élément de liaison (2) s'étendant au moins au voisinage des capuchons de protection oculaire (1) respectivement dans le sens de la largeur (16) des lunettes de protection est rectangulaire,
ii) dans lesquelles l'élément de liaison (2) présente à cet endroit dans le sens de la largeur (16) respectivement une largeur (B) et perpendiculairement à celle-ci dans le sens de la hauteur (17) des lunettes de protection une hauteur (H), dans lesquelles : 1,2≤B/H≤5 s'applique.

2. Lunettes de protection selon la revendication 1, **caractérisées en ce que** le corps de protection inférieur (4) est formé d'un premier matériau et les corps de protection supérieurs (5) sont formés d'un deuxième matériau se distinguant du premier matériau, en particulier en matière de dureté et/ou d'effet protecteur.

3. Lunettes de protection selon la revendication 1 ou 2, **caractérisées en ce que** les corps de protection inférieurs (4) sont souples et sont en particulier faits de silicone.

4. Lunettes de protection selon l'une quelconque des revendications précédentes, **caractérisées en ce que** les corps de protection supérieurs (5) et/ou l'élément de liaison (2) est/sont sensiblement indéformables et est/sont en particulier fait(s) d'une matière plastique thermoplastique, en particulier de polyoxyméthylène.

5. Lunettes de protection selon l'une quelconque des revendications précédentes, **caractérisées en ce que** chaque corps de protection supérieur (5) est relié de manière détachable au corps de protection inférieur (4) associé par coopération de formes et/ou par liaison de matières.

6. Lunettes de protection selon l'une quelconque des revendications précédentes, **caractérisées en ce que** chaque corps de protection supérieur (5) comprend au moins un moyen de liaison par coopération de formes (13) et chaque corps de protection inférieur (4) comprend au moins un moyen de liaison par coopération de formes complémentaire (11) pour la liaison détachable par coopération de formes audit au moins un moyen de liaison par coopération de formes (13) respectif, dans lesquelles ledit au moins un moyen de liaison par coopération de formes (13) est réalisé de préférence sous la forme d'une rainure (13) côté circonférence, en particulier périphérique, et ledit au moins un ou les moyen de liaison par complémentarité de formes complémentaires (11) est réalisé sous la forme d'une nervure, en particulier périphérique.

7. Lunettes de protection selon l'une quelconque des revendications précédentes, **caractérisées en ce que** l'élément de liaison (2) peut être au moins en partie déplacé par rapport aux capuchons oculaires (1) sensiblement perpendiculairement à la direction du regard (10) des lunettes de protection dans le sens de la hauteur (17) de celles-ci.

8. Lunettes de protection selon l'une quelconque des revendications précédentes, **caractérisées en ce que** l'élément de liaison (2) présente à distance des capuchons de protection oculaire (1) une section transversale en particulier rectangulaire, s'étendant longitudinalement dans le sens de la hauteur (17) des lunettes de protection, pour modifier l'écart entre les capuchons de protection oculaire (1).

9. Lunettes de protection selon l'une quelconque des revendications précédentes, **caractérisées en ce que** l'élément de liaison (2) s'étend au moins par endroits dans le sens de la hauteur (17) des lunettes de protection et les lunettes de protection peuvent être portées dans une première position et dans une deuxième position amenée en rotation de 180° par rapport à la première position.

10. Lunettes de protection selon l'une quelconque des revendications précédentes, **caractérisées en ce qu'**un corps d'accouplement (19), faisant en particulier saillie latéralement vers l'extérieur, avantageusement sensiblement indéformable, destiné au maintien, en particulier détachable, d'un dispositif de maintien sur la tête (3), est agencé sur chaque corps de protection supérieur (5).

11. Lunettes de protection selon la revendication 10, **caractérisées en ce que** les corps de protection supérieurs (5) et les corps d'accouplement (9) sont reliés les uns aux autres de manière non détachable, de préférence d'une seule pièce.

12. Lunettes de protection selon la revendication 10 ou 11, **caractérisées en ce que** chaque corps d'accouplement (19) présente au moins au voisinage des capuchons de protection oculaire (1) une section transversale, en particulier rectangulaire, s'étendant longitudinalement dans le sens de la largeur (16) des lunettes de protection, pour le déplacement des corps d'accouplement (19) par rapport aux capuchons de protection oculaire (1) sensiblement perpendiculairement au sens du regard (10) des lunettes de protection dans le sens de la hauteur (17) de celles-ci.

13. Ensemble en tant que constituant de lunettes de protection selon l'une quelconque des revendications précédentes, comprenant
a) deux corps de protection supérieurs (5), qui peuvent être reliés de manière détachable à deux corps de protection inférieurs (4) pour former deux capuchons de protection (1), et
b) un élément de liaison (2) relié aux corps de protection supérieurs (5),
c) dans lequel les corps de protection supérieurs (5) et l'élément de liaison (2) sont reliés les uns aux autres d'une seule pièce et de manière non détachable,
d) dans lequel l'élément de liaison (2) présente au moins au voisinage des corps de protection supérieurs (5) respectivement une section transversale s'étendant longitudinalement dans un sens de la largeur (16) de l'ensemble pour le déplacement de l'élément de liaison (2) par rapport aux corps de protection supérieurs (5) sensiblement perpendiculairement à une direction du regard (10) de l'ensemble dans un sens de la hauteur (17) de celles-ci,
i) dans lequel la section transversale de l'élément de liaison (2), s'étendant au moins au voisinage des corps de protection supérieurs (5) respectivement dans le sens de la largeur (16) de l'ensemble, est rectangulaire,
ii) dans lequel l'élément de liaison (2) présente à cet endroit dans le sens de la largeur (16) respectivement une largeur (B) et, perpendiculairement à celle-ci, dans le sens de la hauteur (17) de l'ensemble, une hauteur (H), dans lequel : 1,2≤B/H≤5 s'applique.
